# EUROPEAN PATENT APPLICATION

(11) **EP 2 042 084 A1**
(43) Date of publication of application: **01.04.2009**
(21) Application number: 08011252.7
(22) Date of filing: 20.06.2008
(51) Int. Cl.: A61B 5/00, A61B 5/117, G06K 9/00

(54) **Electronic appartus having a biometrics authentication**

(30) Priority: 28.09.2007 JP 2007253019
(71) Applicant: Hitachi-Omron Terminal Solutions, Corp., Shinagawa-ku Tokyo 141-0032 (JP)
(72) Inventor: Tsuno, Naoko, Tokyo 100-8220 (JP); Sugiyama, Kenji, Tokyo 100-8220 (JP); Konishi, Yoshiharu, Tokyo 100-8220 (JP)
(74) Representative: Strehl Schübel-Hopf & Partner

(57) **Abstract**

An electronic apparatus having a first structure and a second structure is provided, in which an imaging camera lens along with a two-dimensional sensor is mounted in the first structure and the second structure is provided with an optically transparent opening, whereby a sufficient interval between a finger and the lens to make the camera scan the finger veins with higher quality is secured with the finger placed on the surface of the second structure disposed opposite to the first structure for scanning.

## Description

### Background of the Invention

The present invention relates to an electronic apparatus having a biometrics authentication, in more particularly pertaining to an assembly structure or arrangement of an imaging camera for obtaining biometric information such as finger veins by scanning an image on a finger irradiated with a light source in such as a personal digital assistant.

Conventionally, in order to authenticate an individual, there have been used methods such as the inputting of a code number or a password and the insertion of a magnetic card or a key in a slot. However, as for the former method, the real holders of code numbers and passwords sometimes forget them or others pretend to be the real holders. As for the latter method, cards and keys may be unjustly forged by the third parties. Thus, in recent years, devices for authenticating an individual by use of his/her biometric information have been developed. Among others, there has been known an authentication method to identify a particular individual by use of biometric information on his/her finger veins, which is hard to forge and does not psychologically upset the users.

According to the above method, near infrared light that is irradiated on the finger and internally scattered in the same so as to be externally transmitted, with the result that the light as externally transmitted is taken as an image of biometric information on the finger veins. Incidentally, hemoglobin in the blood appears in shadowy patterns on the taken image because it absorbs such near infrared light, which makes the finger veins relived in the image. The individual authentication is effected by comparing such shadowy patterns with the preliminarily registered patterns of the finger veins through a taken image.

Generally speaking, an imaging camera is in practical use for a device provided with a finger vein authentication function as a tow-dimensional sensor, a lens and so forth being adopted for scanning an image of the finger veins. The prior arts on a scanning sensor of the finger veins as an imaging camera are known from JP-A 2003-298104 and JP-A 2007-156684.

### Summary of the Invention

A conventional imaging camera for scanning the finger veins described in the above references adopts a method of converging an image of the finger into a smaller two-dimensional sensor by means of a lens, which requires the finger as an object for imaging to optically separate from the lens by an interval in order to gain a high-quality focused image thereof. It is therefore necessary to secure an interval between the finger and the lens, which cannot keep an imaging camera including a portion on which the finger is placed from becoming larger in size.

In order to gain a focused image with higher quality for scanning the finger veins, it requires a sufficient interval between the lens and the finger to be secured. The more sufficient the interval is, the larger the imaging camera becomes in size, which does not bring any problems on large devices such as an ATM but causes a problem on the parts mounting space of a personal digital assistant of smaller size such as a cellular phone. The problem to be solved by the invention lies in efficiently mounting an imaging camera for scanning the finger veins on a small-sized personal digital assistant.

In order to solve the above problem, the present invention is characterized in dividing an electronic apparatus for instance a mobile apparatus into a first structure and a second structure, mounting a lens and a two-dimensional sensor of an imaging camera on the first structure, providing an optically transparent opening through the second structure, and disposing a biometric part of an individual on the second structure opposite to the first structure so as to secure an interval between a finger and the lens large enough to scan the finger veins with higher quality.

The invention brings an effect such as an imaging camera being efficiently mounted on the mobile apparatus of smaller size by mounting the camera thereon with the two-dimensional sensor along with the lens and the optically transparent opening to secure an interval between the lens and the biometric part respectively divided into two structures.

### Brief Description of the Drawings

Fig. 1 shows a cross-sectional view of a personal digital assistant in use for authentication of the finger veins according to a first embodiment of the invention;
Fig. 2 shows a cross-sectional view of a personal digital assistant not in use for authentication of the finger veins according to the first embodiment of the invention;
Fig. 3 shows a cross-sectional view of a personal digital assistant in use for authentication according to the second embodiment of the invention;
Fig. 4 shows a trans-sectional view of a personal digital assistant according to the second embodiment of the invention;
Fig. 5 shows a cross-sectional view of a personal digital assistant not in use for authentication of the finger veins according to a second embodiment of the invention;
Fig. 6 shows a trans-sectional view of a personal digital assistant according to a third embodiment of the invention;
Fig. 7 shows a trans-sectional view of a light source for a finger of a personal digital assistant according to the fourth embodiment of the invention; and
Fig. 8 shows a trans-sectional view of an imaging camera of a personal digital assistant according to the fourth embodiment of the invention.

### Best Mode for Carrying out the Invention

A personal digital assistant provided with a function to authenticate finger veins and divided into two structural members is arranged by mounting a two-dimensional sensor and a lens in the first structural member thereof and providing a transparent opening to secure an interval between the finger and the lens as well as light sources to throw light through the finger in the second structural member thereof, wherein those structural elements are disposed therein such that the lens, two-dimensional sensor, transparent opening, and light sources function as one imaging camera for finger veins when the first and second structural members are overlapped one over another.

### (First Embodiment)

Hereinafter, the first embodiment according to the invention is explained.

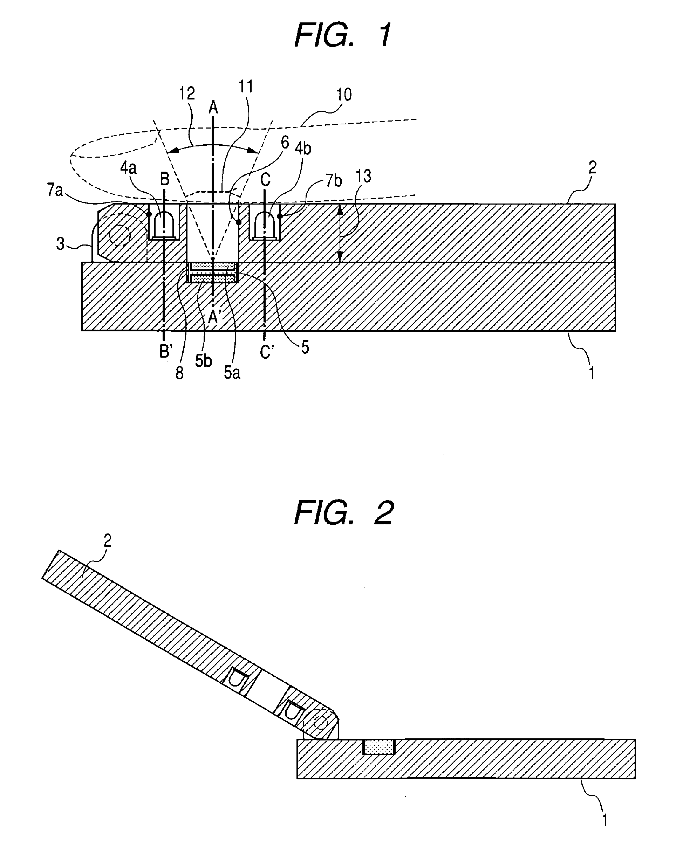

Fig. 1 shows a cross-sectional view of a personal digital assistant in use for scanning a finger image according to the first embodiment of the invention. As shown, a structural member 1 is a structural base for the personal digital assistant and provided with an imaging camera 5 for scanning the finger image and a pivot support member 3 for a hinge mechanism.

A structural member 2 pivots around the support member 3 disposed on the structural member 1, so that those members may be attached together as shown in Fig. 1 or detached from each other as shown in Fig. 2. Light sources 4a and 4b of infrared light emission are mounted in the structural member 2 to irradiate a finger placed on the surface thereof. An optically transparent circular opening 6 for the infrared light is provided through the structural member 2 to scan the irradiated finger image with the imaging camera 5 mounted on the structural member 1.

The respective light sources 4a and 4b are made of infrared light emitting diodes (LED) and provided with luminous intensity and arranged to sufficiently scan the imaging camera 5 with higher quality the finger within a scanning field of vision 12 of the camera 5. The imaging camera 5 essentially includes a lens 5a and a two-dimensional sensor 5b, and scans an interior image of the finger including its veins pattern 11 with the finger image within the scanning field of vision 12 converged through the lens 5a into the two-dimensional sensor 5b. The transparent opening 6 is provided through the structural member 2, so that the length of the opening 6 is has the same as thickness 13 of the structural member 2.

The thickness 13 of the structural member 2 or the length of the opening is defined such that the imaging camera 5 makes an optically optimum interval with the finger so that it can take the finger interior image including its veins pattern 11 with higher quality. As shown in Fig. 1, the scanning and authentication steps are taken with the structural members 1 and 2 attached together, in which no light penetrates into the camera 5 from an interstice between them and the optical axis of the imaging camera corresponds to the central axis of the cylindrical opening 6 so as to make the same axial line A-A' between them, therefore those members are disposed to each other in an optimum position to scan the finger image.

According to the present embodiment, it is necessary to secure a sufficient interval between the camera lens and the finger to scan an image of the finger veins with higher quality, which unavoidably makes the camera large in size to such an extent that it is rendered inappropriate for being mounted into a personal digital assistant of smaller size. However, it has a merit in that the imaging camera may be efficiently mounted in a digital assistant by making use of its structure.

### (Second Embodiment)

The first embodiment shows the structural member 2 pivot around the support member of the structural member 1 with a hinge mechanism provided between those members. According to the present embodiment, a sliding mechanism is provided between those members, by which the structural member 2 moves parallelwise with regard to the structural member 1.

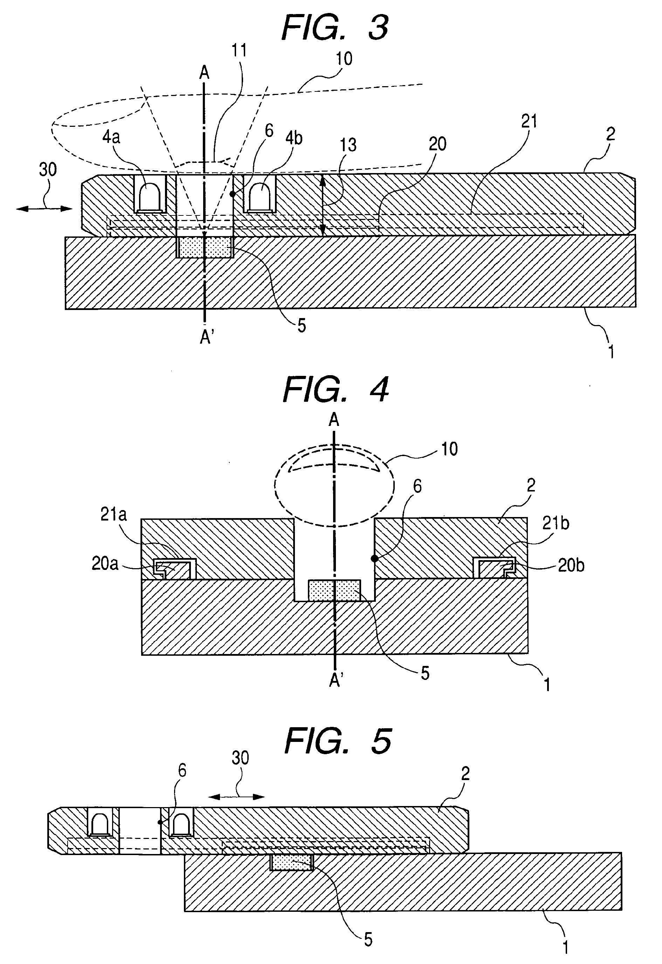

Fig. 3 shows a cross-sectional view of a personal digital assistant according to the second embodiment scanning a finger image. Fig. 4 shows a trans-sectional view of the assistant taken along the optical centerline A-A' of the imaging camera. Apart from the sliding mechanism disposed between those members 1 and 2, the imaging camera 5, the light sources 4a and 4b as well as the cylindrical opening 6 hereof are the same as those of the first embodiment.

As shown in Fig. 4, the structural member 1 is provided with sliding rails 20a and 20b, so that the structural member 2 slides on the surface of the structural member 1 to the direction 30 as shown in Fig. 3. In Fig. 5, the structural member 2 moves by way of the sliding mechanism to the left side with respect to the drawing along the direction 30 and comes to stop at the farthest left end of the structural member 1. In Fig. 3, the structural member 2 moves along the direction 30 and comes to stop at the farthest right end thereof, at which the optical axis of the imaging camera 5 corresponds to the central axis of the cylindrical opening 6 through the same axial line A-A', so that those members 1 and 2 are placed in an optimum position to scan the finger image for authentication.

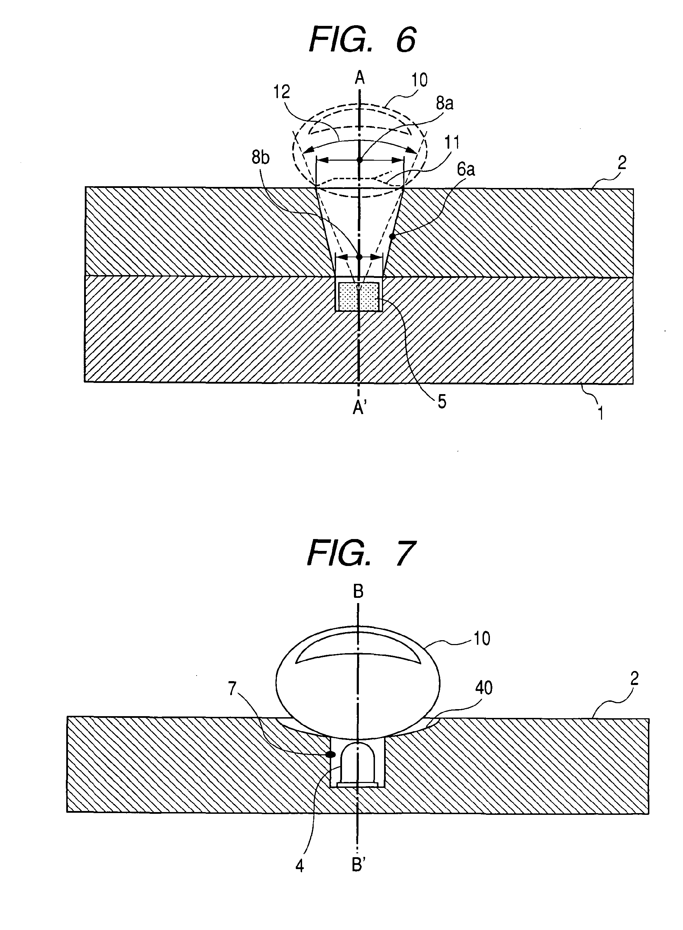

In the same way as the first embodiment, the present embodiment has a merit in that the imaging camera may be efficiently mounted into a personal digital assistant by making use of its structure. (Third Embodiment) The difference between the present embodiment and the first and second embodiments respectively lies in the opening 6 hereof taking the shape of a truncated cone instead of the cylindrical shape adopted in the above embodiments. Fig. 6 shows a trans-sectional view of a personal digital assistant in use for scanning the finger image according to the present embodiment.

A scanning field of vision 12 of the imaging camera 5 as shown in Fig. 6 is an area over which light irradiated from the finger and reaching the camera 5 passes when the finger veins 11 is scanned. The opening 6 of the structural member 2 as shown in the first embodiment does not have to be cylindrical in shape but may be a truncated cone as shown with 6a, with its shape of a circle of a diameter 8a on the upper surface of the structural member 2 and its shape of a circle of a diameter 8b on the lower surface the structural member 2. According to the present embodiment, the volume of the transparent opening of the structural member 2 can be reduced, allowing more efficient use of the mounting area of the personal digital assistant.

### (Fourth Embodiment)

The present embodiment is the same as the first and second embodiments as mentioned above, except that a portion of the structural member 2 on which a finger is placed is concavely recessed. Fig. 7 shows the present embodiment, in which a trans-sectional view of the structural member 2 is taken along the B-B' line or the C-C' line centered on the light source as shown in Fig. 1 showing the cross-sectional view of a personal digital assistant according to the first embodiment.

As shown, a recessed portion 40 is provided on the surface of the structural member 2. When a finger 10 is placed in the recessed portion, the finger ball completely closes an entrance of a hole 7 in which a light source 4 is mounted, so that light emitted from the light source 4 does not leak directly from the structural member 2, and reaches outside internally passing through the finger 10. Thus, the finger 10 is internally irradiated with the light source 4 in an efficient manner.

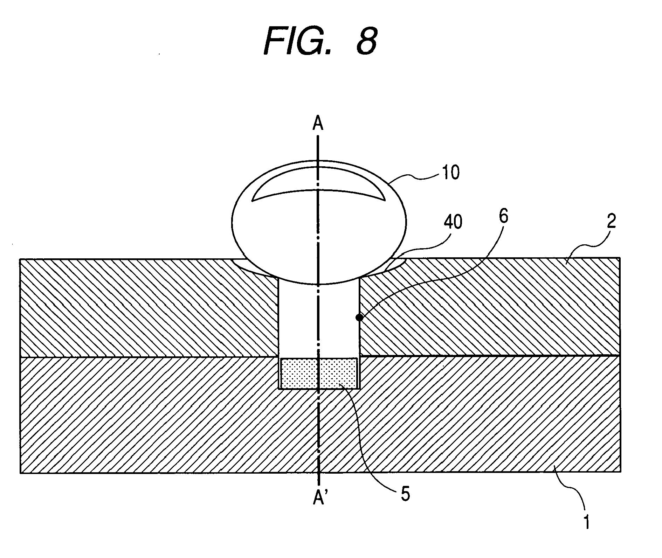

Fig. 8 shows a trans-sectional view of the first and second structural members 1 and 2 taken along the A-A' line of Fig. 1. In the same way as the light source as shown in Fig. 7, the finger ball completely closes the entrance of the opening 6 of the structural member 2. Thus, the imaging camera 5 scans no incident light penetrating from the outside of the personal digital assistant, but scans only light irradiated from the finger.

The arrangement of the present embodiment is explained above with reference to that of the first embodiment, but it is also applicable to that of the second embodiment.

According to the present embodiment, the provision of the concave configuration in a portion of the personal digital assistant on which the finger is mounted allows light emitted from the light source to internally penetrate into the finger in an efficient manner and the finger image to be scanned with high quality without incident light deriving from the outside of the digital assistant.

## Claims

1. An electronic apparatus comprising:
a first structure in which an imaging camera to internally scan an image on the finger irradiated with a light source is assembled therein, and
a second structure being engaged with the first structure, in which the light source for irradiating a light having a predetermined wavelength is assembled therein,
wherein the second structure is provided between the imaging camera and the finger with an optically transparent opening dimensionally defined such that the imaging camera keeps an optimum interval for imaging with the finger.

2. The electronic apparatus according to claim 1,
wherein said electronic apparatus is a personal digital assistant including the first structure provided with a pivot support member for a hinge mechanism and the second structure to pivot around the support member, the first and second structures capable of being foldably overlapped one over another through the hinge mechanism, and
wherein, with the first and second structures overlapped one over another, the light source is mounted with regard to the finger in the second structure disposed oppositely to the first structure to internally irradiate the finger in contact with a surface of the second structure.

3. The electronic apparatus according to claim 1,
wherein said electronic apparatus is a personal digital assistant including the first structure and the second structure to relatively change a position through a predetermined sliding mechanism with regard to the first structure, and the first and second structures being modifiable in shape so as to be foldably overlapped one over another through the sliding mechanism, and
wherein, with the first and second structures overlapped one over another, the light source is mounted with regard to the finger in the second structure disposed oppositely to the first structure to internally irradiate the finger in contact with a surface of the second structure.

4. The electronic apparatus according to claim 2,
wherein the transparent opening of the second structure takes a shape of a truncated cone and a circle of a side of the second structure in contact with the first structure is smaller in diameter than a circle of an opposite side of the second structure.

5. The electronic apparatus according to claim 3,
wherein the transparent opening of the second structure takes a shape of a truncated cone, whereby a circle of a side of the second structure in contact with the first structure is smaller in diameter than a circle of an opposite side of the second structure.

6. The electronic apparatus according to claim 2,
wherein a surface of the second structure, on which the finger is placed, is concave in shape, and structurally arranged such that a hole entrance of the light source provided on the surface to irradiate the finger and an opening entrance provided on the surface to continue to the opening for making the imaging camera scan the finger are closed with balls of the finger when the finger is placed so as to block external light from entering.

7. The electronic apparatus according to claim 3, wherein a surface of the second structure, on which the finger is placed, is concave in shape, and structurally arranged such that a hole entrance of the light source provided on the surface to irradiate the finger and an opening entrance provided on the surface to continue to the opening for making the imaging camera scan the finger are closed with balls of the finger when the finger is placed so as to block external light from entering.
